(19) Europäisches Patentamt
European Patent Office
Office européen des brevets

(11) Publication number : **0 540 185 A1**

# (12) EUROPEAN PATENT APPLICATION

(21) Application number : **92309053.4**

(22) Date of filing : **05.10.92**

(51) Int. Cl.$^5$ : **C07D 498/22,** A61K 31/55, // (C07D498/22, 311:00, 273:00, 209:00, 209:00, 209:00)

(30) Priority : **10.10.91 US 774395**

(43) Date of publication of application : **05.05.93 Bulletin 93/18**

(84) Designated Contracting States : **PT**

(71) Applicant : **SCHERING CORPORATION**
**2000 Galloping Hill Road**
**Kenilworth New Jersey 07033 (US)**

(72) Inventor : **Kirkup, Michael P.**
**753 Cedarbrook Road**
**Bridgewater, New Jersey 08807 (US)**

(74) Representative : **Ritter, Stephen David et al**
**Mathys & Squire 10 Fleet Street**
**London EC4Y 1AY (GB)**

## (54) 4'-(N-substituted-N-oxide)staurosporine derivatives.

(57) A compound represented by formula I

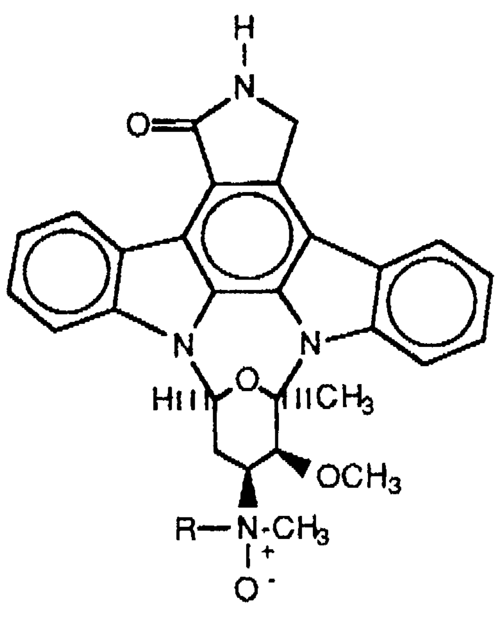

I

wherein R is a ($C_1$-$C_{10}$) alkyl, e.g., $CH_3$ or an arylmethyl group, e.g. benzyl and N-oxide stereochemical isomers thereof or a pharmaceutically acceptable salt thereof as well as pharmaceutical compositions containing a compound of formula I and a method of treating tumors or psoriasis using a compound of formula I or a pharmaceutical composition thereof are disclosed.

EP 0 540 185 A1

BACKGROUND

This invention relates to 4′-(N-substituted-N-Oxide)staurosporine derivatives and pharmaceutical compositions containing such derivatives and method of treating susceptible tumors in mammals in need of such treating.

Various indolocarbazoles are known. The indolocarbazole, staurosporine, is an alkaloid compound produced by fermentation of a suitable microorganism belonging to the genus Streptomyces. A suitable strain for staurosporine, production is Streptomyces AM-2282, NRRL 11184 which is disclosed in USP 4,107,297. Staurosporine has antibiotic activity in yeast and fungi and has a therapeutic effect on hypertension, edema and ulcers in rats. N-acetylstaurosporine is disclosed and claimed in Australian Patent Application -17571/88 by CIBA-Geigy on December 15, 1988 as a N-substituted derivative of staurosporine being capable of selective modification of the activity of protein kinase C ("PKC") and as such can be useful for the preventive or curative treatment of disease in which the inhibition of PKC is of importance in a warm blooded animal as well as can be employed as medicaments for tumor-inhibition, inflammation-inhibition, immunomodulation and also in preparations for combating bacterial infections, arteriosclerosis, as well as other diseases of the cardiovascular system and of the central nervous system.

T. Tamaoki et al. disclose in Biotechnology, Vol. 8 (August 1990) 732-736 that the indolocarbazole, UCN-01 shows selectivity for PKC inhibition and has in vitro antitumor activity against human tumor cells including cervical cancer HeLa S3 cells, bladder carcinoma T-24 cells, colon adenocarcinoma COLO 320DM cells and against P388 murine leukemias in vivo and against xenograft models cells transformed with oncogenes, and that straurosporine does not appear to have antitumor activity at non-toxic levels in the in vivo models.

Staurosporine and UCN-01 are represented by the structural formula S:

S

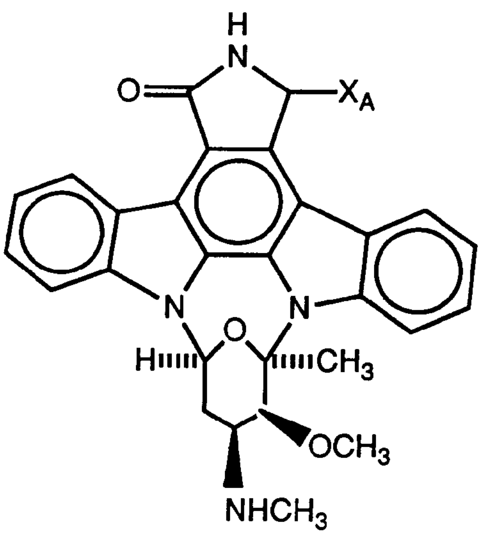

staurosporine: $X_A$ = H
UCN-01 : $X_A$ = OH

T. Tamaoki et al. supra, disclose that these results suggest that selectivity for PKC inhibition by UCN-01 may correlate with the in vivo antitumor effects by UCN-01, whereas the potent but non-selective inhibition for protein kinases exhibited by staurosporine can not.

There is a need for antitumor compounds which effectively and safely treat mammals.

**SUMMARY OF THE INVENTION**

The present invention provides compounds represented by the formula I:

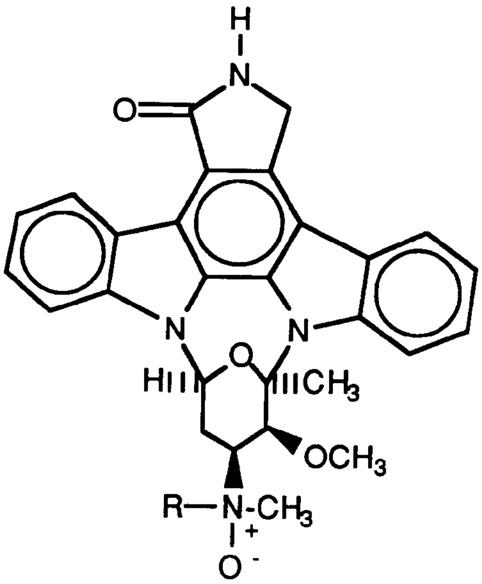

I

wherein R is a straight or branched chain ($C_1$-$C_{10}$) alkyl group or an arylmethyl group and the nitrogen oxide sterechemical isomers thereof.

The present invention also provides pharmaceutical compositions for treating tumors or psoriasis comprising an effective amount of a compound of formula I for each of such purposes and a pharmaceutically acceptable carrier.

The present invention also provides a method of treating tumor in mammals which comprises administering to a mammal in need of such treating an antitumor amount of a compound of formula I effective for such purpose or a pharmaceutical composition containing said effective amount of said compound.

The present invention also provides a method for treating psoriasis in mammals which comprises administering to a mammal in need of such treating an antipsoriatically effective or a pharmaceutical composition containing such effective amount of said compound.

## DETAILED DESCRIPTION OF THE INVENTION AND OF THE PREFERRED EMBODIMENTS

The compounds of formula I wherein R is not methyl exist as steroisomers, i.e., diastereoisomers. Staurosporine represented by formula S($X_a$=H) has four chiral centers. A new chiral center is introduced into the staurosporine molecule at the 4′ nitrogen by formation of the 4′-N-substituted N oxides of staurosporine represented by formula I ($R \neq CH_3$). See, for example, Example 2 wherein the diastereoisomers of 4′-N-n-butyl-N-oxide of staurosporine are prepared and isolated.

The term "($C_1$-$C_{10}$) alkyl group" means $C_1$-$C_{10}$ straight and branched chain alkyl groups including methyl, ethyl, n and iso-propyl, n,- iso,-sec, and tert-butyl, n,-iso, -sec,-tert and neo-pentyl, -hexyl, -heptyl, -octyl, -nonyl and decyl groups.

The term "arylmethyl" means benzyl, 1-naphthylmethyl, 2-naphthylmethyl, indanylmethyl, i.e., substituted -1-naphthylmethyl, -2-naphthylmethyl, -indanyl, -benzyl wherein the substituents on naphthyl, indanyl or phenyl of benzyl are selected from one two or three of ($C_1$-$C_{10}$) alkyl, ($C_1$-$C_{10}$) alkoxyl, halogen, hydroxyl, nitro, N,N-di($C_1$-$C_{10}$) alkylamino and ($C_1$-$C_{10}$) alkyl substituted by halogen, hydroxy and ($C_1$-$C_{10}$) alkoxy. Exemplary arylmethyl groups include benzyl, 4-hydroxybenzyl, and 4-N,N-dimethylaminobenzyl.

The term "($C_1$-$C_{10}$) alkoxy" means ($C_1$-$C_{10}$) alkyl groups univalently bonded to divalent oxygen, -O- e.g. methoxy, ethoxy.

The term "halogen" means, F, Cl, Br and I.

The term "($C_1$-$C_{10}$) alkyl substituted by halogen, hydroxy and ($C_1$-$C_{10}$) alkoxy" include ($C_1$-$C_{10}$) alkyl groups substituted by one or more halogens such as perhaloalkyl, e.g. trichloromethyl, trifluoromethyl as well as chloromethyl, ($C_1$-$C_{10}$) alkyl groups substituted by hydroxy or ($C_1$-$C_{10}$) alkoxy including hydroxy methyl and methoxy methyl or methoxyethyl.

The compounds of formula I may be prepared from staurosporine by use of Scheme A

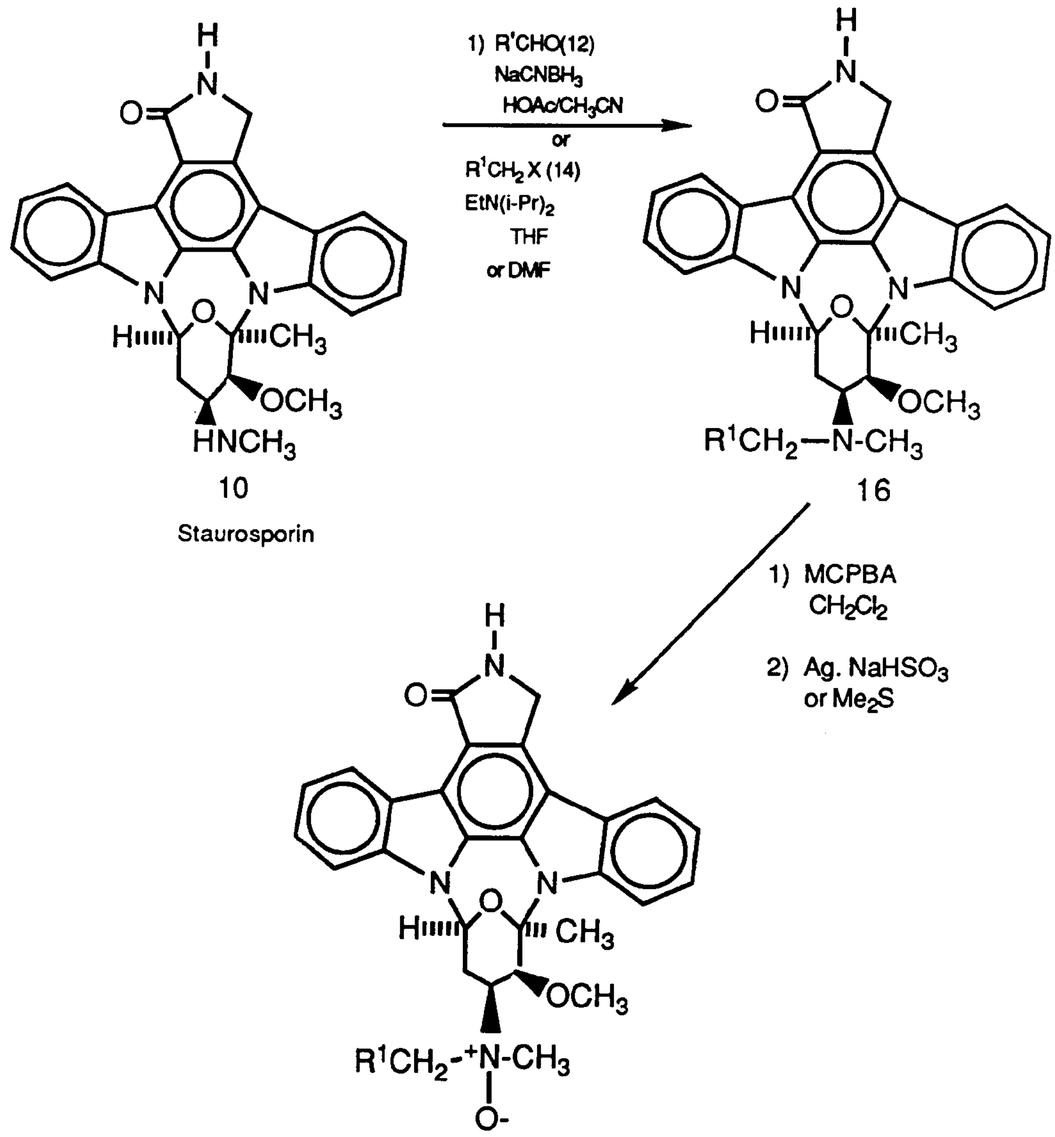


10

Staurosporin

16

I

Staurosporine 10 was dissolved in an inert solvent such as acetonitrile ($CH_3CN$) is reacted with aldehyde 12, R'CHO and glacial acetic acid. Sodium cyanoborohydride ($NaCNBH_3$) is then added in portions. The temperature of the reductive amination is usually about 0°- 60°C and reaction time varies from about 1 hour to several days. Other reducing agents such as t-butylamine borane suitable for reduction of the imine bond, but not the carbonyl aldehyde, moiety may also be used. The R′ in the aldehyde R′CHO maybe H or a ($C_1$-$C_9$) straight or branched chain alkyl group. Such aldehydes are commercially available or made from the commercially available alcohols or carboxylic acids using conventional oxidation or reduction reagents. The conversion of staurosporine into N-alkyl staurosporine 16 may also be accomplished by alkylation of 10 with alkyl halide $R'CH_2X$, 14, disolved in an aprotic solvent such as DMF or THF in the presence of a base. In $R'CH_2X$, R′ is H or a ($C_1$-$C_9$) straight or branched chain alkyl group and X is halo, preferably Br or I. The preferred base is an organic tertiary amine such as ethyl di-iso-propylamine in amounts sufficient to react with the acid, HX, produced in the alkylation reaction. The alkylation reaction temperature varies from about 0°C to the reflux temperature and the reaction time varies from about 1 hr to several days. Compound 16 is converted into the N-oxide compound 18 by use of a peracid such as m-chloroperoxybenzoic acid ("MCPBA") or other per acids such as peroxyacetic acid or trifluoroperoxyacetic acid. The excess peroxyacid is quenched/destroyed by addition to the reaction mixture of dimethylsulfide or aqueous sodium hydrogen sulfite.

Purification of the crude product involves standard separation and chromatographic techniques to provide

compounds of formula 18 wherein $R'CH_2$ in 18 is equivalent to R in compound of formula I.

The compounds of formula I of the invention are useful as agents for treating tumors. For example, they are useful as agents in the treatment of tumors of the skin, colon and breast. The compounds of the invention reduce colony formation in HT-24 human breast carcinoma and HT-29 human colon carcinoma cells.

Moreover, Meyer et al, Int. J. Cancer, 43, 851-856 (1989) and Takahashi et al, The Journal of Antibiotics, Vol. XL, No.. 12, 1782-1783 (1987) have recently demonstrated that other compounds which are indolocarbazoles and which inhibit PKC also inhibit murine lymphotic leukemia P388 as well as HL-60 human promyelocytic leukemia. On the basis of the two publications mentioned just above, the compounds of the invention are expected to inhibit the P388 and HL-60 leukemia cell lines.

The potential for compounds of formula I of the invention to have anti-tumor activity, may be demonstrated by their ability to inhibit protein Kinase C ("PKC") in vitro as shown in the test protocol just below.

## TEST METHODS

The below listed Test Methods A and B were run in accordance with the procedures described in Bishop, W.R., Petrin, J., Wang, L., Ramesh, U., and Doll, R.J. (1990) Biochemical Pharmacology, Vol. 40,2129-2135 and Hannun et al. J. Biol. Chem. (1985), Vol. 260, 10039-10043, which are herein incorporated by reference with the following modifications.

### A. **Protein Kinase C ("PKC") Protocol**

Mixed micellular protein kinase C assays were conducted in accordance with the methods described in the two articles cited just above (namely, Bishop et al, and Hannun et al), in a reaction mixture (0.25 mL total volume) containing: 20 mM Tris-HCl (pH 7.5); 200 mg/mL histone III-S; 10 mM $MgCl_2$; 5 mM [g-32P]ATP (4 x 106 cpm/mmol); 200 mM $CaCl_2$; 32 mg/mL of phosphatidylserine; 1.6 mg/mL of 1-oleoyl-2-acetylglycerol and various concentrations of compounds of this invention added from a dimethyl-sulfoxide (DMSO) stock solution. The final concentration of DMSO in the reaction was 2% w/v. Reactions were started by addition of approximately 7 mg of partially purified rat brain PKC and allowed to proceed at 23°C for 15 min. Reactions were terminated by collection of trichloroacetic acid-precipitated phosphorylated histone on GF/C filters using a Brandel Cell Harvester (Model No. M-24-R).

### B. **Myosin Light Chain Kinase ("MLCK") Protocol**

Myosin light chain kinase (MLCK) assays were conducted in accordance with the methods of the Bishop et al. and Hannun et al. articles listed above in a reaction mixture containing 30 mM MLCK substrate peptide (Lys-Lys-Arg-Pro-Gln-Arg-Ala-Thr-Ser-Asn-Val-Phe-Ser-$NH_2$), 10 nM calmodulin, 25 mM [g-32P]ATP; 0.1 mM $Ca^{+2}$ acetate and 10 mM $Mg^{+2}$ acetate. Reactions were stopped after 10 minutes by addition of 100 mL of 375 mM $H_3PO_4$ and spotting on phosphocellulose P81 paper.

The following criteria were used to determine desired potency and/or selectivity: compounds with a PKC $IC_{50}$ value of less than 100 nM or a PKC $IC_{50}$ value of less 5 μM and compounds with a selectivity (MLCK/PKC) of greater than 10 are considered more active.

## RESULTS

IC50 (mM inhibition)

| Example | R in formula I | MLCK | PKC | MLCK/PKC |
|---|---|---|---|---|
| 1 | $CH_3$ | 34 nM | 3.1 nM | 11 |
| 11 | $C_6H_5CH_2$ | 55 | 33 | 16.7 |
| 2 | n $C_4H_9$[1] | 200 | 30 | 6 |
| 2 | n $C_4H_9$[1] | 245 | 18 | 13.6 |

1) Diastereoisomers see Example 2

The anti-psoriatic activity of the compounds of formula I of the present invention may be demonstrated by use of the following experimental protocol.

**C. Inhidition of Spontaneous and Induced Cornified Envelope ("CE") Formation in Normal Human Epidermal Keratinocyte (NHEK) Cultures.**

The data was collected in cultured NHEK cells which were grown in serum-free media to subconflurncy and then treated with DMSO (vehicle control) or various concentration of the compounds of this invention, etretinate (an antipsoriatic aromatic analog of retinoic acid) and staurosporine.

After 15 minutes, the NHEK cells were treated with DMSO (vehicle control), 200 mg of 12-tetradecanoylphorbol-13-acetate ("TPA") to evaluate TPA-induced CE formation. In another experiment, the NHEK cells were refed media containing 0.15 mM $Ca^{+2}$ (control) or a high calcium-containing media, i.e., a 1.6 mM $Ca^{+2}$ media to induce CE formation by such a high calcium-containing media. After 48 hours, the cells so-treated were harvested and viable and total cells were counted. Cornified envelopes were assessed after resuspending cells pellets in buffer containing sodium dodecyl sulfate ("SDS") and dithiothreitol ("DTT"). CE formation induced by TPA or high calcium-containing media was expressed as CE/viable cells and each treatment group was compared to its appropriate control group.

The compounds of formula I inhibit cornified envelope formation in human keratinocytes. For example, the preferred compound of formula I wherein R=benzyl, exhibited (1) approximately 90% inhibition of envelope formation in this test model at a concentration of 250 mM (2) 71% and 74% CE formation induced by high calcium-containing media at 500 and 1000 mM concentrations, respectively; and (3) antagonizes TPA-induced CE formation at a concentration of 250 mM.

Staurosporine (at a concentration of <10 mM) potently inhibited CE formation in this test model but cytotoxicity was observed at even such low concentrations.

The inhibition of keratinocyte cornification in this test model is a reproducible effect of the antipsoriatic etretinate, an aromatic analog of retinoic acid. Accordingly, inhibition of CE formation by the compounds of this invention in this test model is expected to predict antipsoriatic activity in mammals, especially man.

A pharmaceutical composition of the invention comprises a therapeutically effective amount of a compound of the invention in combination with a pharmaceutically acceptable carrier material.

The compounds of this invention can be administered in any number of conventional dosage forms, e.g., topical, oral, parenteral, rectal, transdermal, and the like. Oral or rectal dosage forms include capsules, tablets, pills, powders, cachets, and suppositories. Liquid oral dosage forms include solutions and suspensions. Parenteral preparations include sterile solutions and suspensions. Topical dosage forms can be creams, ointments, lotions, transdermal devices (e.g., of the conventional patch or matrix type) and the like.

The formulations and pharmaceutical compositions contemplated by the above dosage forms can be prepared with conventional pharmaceutically acceptable excipients and additives, using conventional techniques. Such pharmaceutically acceptable excipients and additives are intended to include carriers, binders, flavorings, buffers, thickeners, coloring agents, stabilizing agents, emulsifying agents, dispersing agents, suspending agents, perfumes, preservatives lubricants, etc.

Suitable pharmaceutical acceptable solid carriers are magnesium carbonate, magnesium stearate, talc, sugar, lactose, pectin, dextrin, starch, gelatin, tragacanth, methylcellulose, sodium carboxymethylcellulose, low melting waxes, cocoa butter and the like. Capsules can be made wherein the active compound is inserted into the capsules along with a pharmaceutically acceptable carrier. The active compounds of this invention can be mixed with pharmaceutically acceptable excipients or be used in finely divided powder form without excipients for inclusion into the capsules. Similarly, cachets are included.

Liquid form preparations include solutions, suspensions and emulsions such as water or water-propylene glycol solutions for parenteral injection. Liquid preparations can also be formulated in solution in polyethylene glycol and/or propylene glycol, which may contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the active component in finely divided form in water with viscous material, i.e., pharmaceutically acceptable natural or synthetic gums, resins, methylcellulose, sodium carboxymethylcellulose and other well-known suspending agents.

Formulations for topical application may include the above liquid forms, as well as creams, aerosols, sprays, dusts, powders, lotions and ointments which are prepared by combining an active ingredient according to this invention with conventional pharmaceutical acceptable diluents and carriers commonly used in topical, dry, liquid, cream and aerosol formulations. Ointment and creams may, for example, be formulated with an aqueous or oily base with the addition of suitable thickening and/or gelling agents. Such bases may, thus, for example, include water and/or an oil such as liquid paraffin or a vegetable oil such as peanut oil or castor oil. Thickening agents which may be used according to the nature of the base include soft paraffin, aluminum stearate, cetostearyl alcohol, propylene glycol, polyethylene glycols, woolfat, hydrogenated lanolin, beeswax, etc.

Lotions may be formulations with an aqueous or oil base and will, in general, also include one or more of pharmaceutically acceptable stabilizing agents, emulsifying agents, dispersing agents, suspending agents, thickening agents, coloring agents, perfumes and the like.

Powders may be formed with the aid of any suitable pharmaceutically acceptable powder base, e.g., talc, lactose, starch, etc. Drops may be formulated with an aqueous base or non-aqueous base also comprising one or more pharmaceutically acceptable dispersing agents, suspending agents, solubilizing agents, etc.

The topical pharmaceutical compositions may also include one or more preservatives or bacteriostatic agents, e.g., methyl hydroxybenzoate, propyl hydroxybenzoate, chlorocresol, benzalkonium chlorides, etc.

The topical pharmaceutical compositions may also contain an active compound of this invention in combination with other active ingredients such as antimicrobial agents, particularly antibiotics, anesthetics, analgesics and antipruritic agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses under conditions which retard possible decomposition. The solid form preparations intended to be converted to liquid form may contain, in addition to the active material, pharmaceutically acceptable flavorants, colorants, stabilizers, buffers, artificial and natural sweeteners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

The compounds of this invention may also be deliverable transdermally for systemic distribution. The transdermal compositions can take the form of creams, lotions and/or emulsions and can be included in a transdermal patch of the matrix or reservoir type as are conventional in the art for this purpose.

The dosage range of the compounds of the invention varies from about 0.1 mg/kg to about 100 mg/kg of body weight, preferably about 1 to 50 mg/kg of body weight per day.

The compounds of this invention may be administered by any conventional mode of administration by employing a therapeutically effective amount of a compound of this invention for such mode. The dosages may be varied depending upon the age, sex, body weight the individual condition and requirements of the patient in the judgment of the attending clinician, the severity of the condition being treated and the particular compound being employed. Determination of the proper dosage for a particular situation is within the skill of the attending clinician. Treatment can be initiated with smaller dosages which are less than the optimum dose of

a compound of this invention. Thereafter, the dosage should be increased by small increments until the optimum effect under the circumstances is reached. For convenience, the total daily dosage may be divided and administered in portions during the day if desired.

The following examples illustrate the present invention; the scope is defined by the claims.

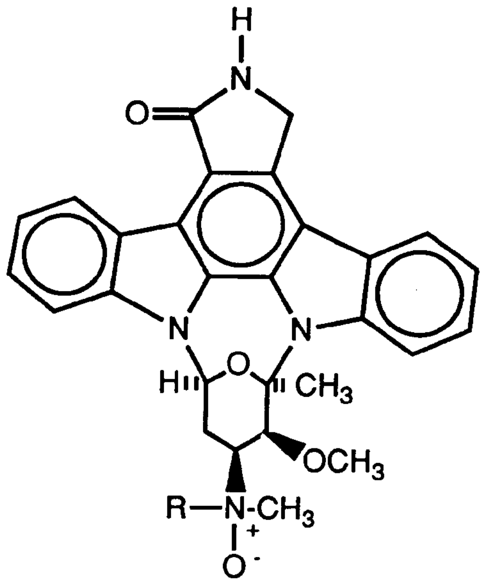

I

| Example No. | R |
|---|---|
| 1 | $CH_3$ |
| 2 | $C_4H_9$ (2 diastereoisomers) |
| 3 | $C_2H_5$ (2 diastereoisomers) |
| 4 | $C_3H_7$ (2 diastereoisomers) |
| 5 | $C_5H_{11}$ (2 diastereoisomers) |
| 6 | $C_6H_{13}$ (2 diastereoisomers) |
| 7 | $C_7H_{15}$ (2 diastereoisomers) |
| 8 | $C_8H_{17}$ (2 diastereoisomers) |
| 9 | $C_9H_{19}$ (2 diastereoisomers) |
| 10 | $C_{10}H_{21}$ (2 diastereoisomers) |
| 11 | $C_6H_5CH_2$- (2 diastereoisomers) |
| 12 | 4-$CH_3OC_6H_4CH_2$- (2 diastereoisomers) |
| 13 | 4-$CF_3$-$C_6H_4$-$CH_2$- (2 diastereoisomers) |
| 14 | 4-$(CH_3)_2$-N-$C_6H_4$-$CH_2$ (2 diastereoisomers) |
| 15 | 4-$CH_3$-$C_6H_4$-$CH_2$- (2 diastereoisomers) |
| 16 | 2,4-$(CH_3)_2C_6H_4CH_2$- (2 diastereoisomers) |

Example 1

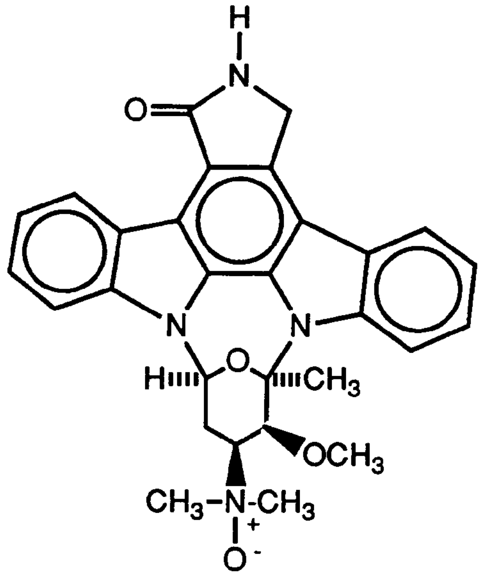

I

A) To a suspension of staurosporin (50 mgs, 0.11 mmols) in acetonitrile (3 mL) was added 4 drops of 37% aq. formaldehyde (52 mgs) and 30 drops of glacial acetic acid, followed by cooling of the reaction mixture to 10°C and then portion wise addition of solid sodium cyano-borohydride (100 mgs over 10 min). An additional 30 drops of glacial acetic acid were added and the solution was kept at room temperature for one hour. Then 3 mL of 2 N NaOH was added and, after 15 minutes, the mixture was diluted with 5 mL of water and the product was extracted into diethylether (2 x 10 mL), dried ($MgSO_4$) and concentrated under vacuum to a colorless glass. Product was purified by preparative HPLC (10% $MeOH/CH_2Cl_2$) to provide 23 mgs of N-methylstaurosporine : PNMR:δ ($CDCl_3$): 2.09 (s,3H); 2.50(s,3H); 2.61 (s,3H); 2.89(s,3H); 2.6(m, obscured) 3.04(dt, 1 H, J=3.5, 10 $H_z$); 4,65 (s, 1H); 4.90 and 4.98 (two doublets, 2H, J=14$H_z$);6.62 (broad s, 1H, $D_2O$ exchangeable), 6.78 (dd, 1H, J=14.5 $H_z$); 7.38 (two triplets, 2H, J=9.5 $H_z$); 7.49 (two triplets, 2H, J=10 $H_z$), 7.84 (overlapping doublets, 2H, J=10 $H_z$), 9.45 (d, 1H, J=9.6 $H_z$).

FAB MS m/z = 481 (M+1)

B) The N-methyl staurosporine (42 mgs 0.09 mmoles) of Example 1A was dissolved in 2 mL of $CH_2Cl_2$ and the so-formed solution cooled to 5°C in a ice bath. A solution of m-chloroperoxybenzoic acid (MCPBA) (35 mgs) was added thereto with stirring. The reaction was complete in under 30 seconds and excess MCPBA was quenched with 2-3 drops of dimethylsulfide. The so-formed mixture was extracted with dilute aqueous sodium bicarbonate and then water. The precipitated product was removed by filtration. The mother liquor yielded additional product after concentration and purification by preperative HPLC (20% $MeOH/CH_2Cl_2$) to provide 23 mg of 4′ N-methyl-N-oxide of staurosporine. PNMR:δ ($CDCl_3$); 2.20 (s, 3H); 2.53 (s, 3H); 2.66 (t, 1H, J=6.5Hz); 3.0 (s, 3H), 3.11 (m, 1H); 3.37 (s, 3H); 3.91 (m, 1H); 5.0 (s, 2H); 5.03 (s, 1H) 6.32 (br. singlet, 1H, $D_2O$ exchangeable); 6.80 (dd, 1H, J=6.5, 2$H_z$); 7.22 (d, 1H, J=9.5 $H_z$); 7.40 (2 doublets, 2H, J = 9.5 $H_z$), 7.51 (2 doublets, 2H, J=10.5 $H_z$), 7.71 (d, 1H, J=10.0 $H_z$); 7.94 (d, 1H, J=9.5 $H_z$); 9.48 (d, 1H, J=10.5 $H_z$) Molecular Formula by

High Resolution MS: $C_{29}H_{29}N_4O_4$ (M $^+$1)

Molecular Weight:

Calc: 497.2189

Meas.: 497.2180

Example 2

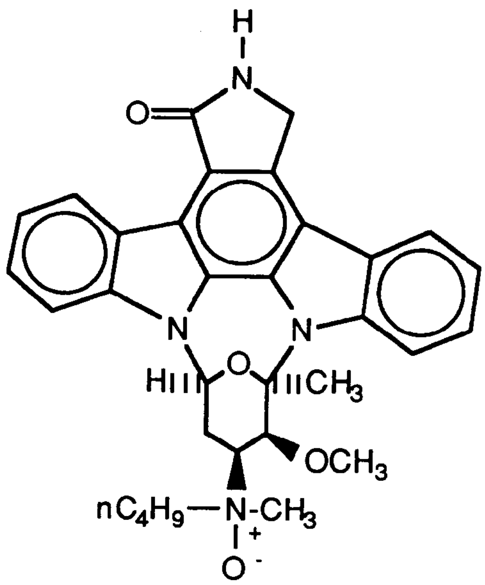

I

A. To a solution of staurosporine (30 mgs, 0.066 mmoles), in 2 mL acetonitrile, was added a solution of n-butyraldehyde in acetonitrile (1 mL). Then 10 drops of glacial acetic acid were added followed by portion-wise addition of sodium cyanoborohydride (20 mgs). An additional 20 drops of acetic acid were added and the so-formed reaction mixture was stirred at room temperature for 30 minutes. Then, 4 mL of in NaOH was added and the so-formed reaction mixture was stirred for 15 minutes. The reaction mixture was then diluted with 5 mL of water and product was extracted into ethyl acetate (2 x 10 mL, and the organic layer was dried ($MgSO_4$) and concentrated. The product was purified by preparative HPLC (8% $MeOH/CH_2Cl_2$) to give 25 mgs of 4-N-(n-butyl)staurosporine.

PNMR $\delta$ ($CDCl_3$): 0.75 (t, 3H, J=6.2 $H_z$); 1.0-1.2 (m, 4H); 2.02 (s, 3H); 2.25 (m, 1H); 2.40 (m, 1H, obscure by methyl signal); 2.40 (s, 3H); 2.50 (s, 3H); 2.60 (t, 2H, J = 6.5 $H_z$); 3.19 (t, 1H, J = 5.5 $H_z$); 3.49 (s, 3H); 3.91 (s, 1H); 4.95 (2 doublets, 2H, J=12.2 $H_z$); 6.6 (dd, 1H, J=5.5, 2$H_z$); 6.8 (br. s, 1H, exchangeable with $D_2O$); 7.21 (d, 1H, J = 9.5 $H_z$); 7.33 (2 doublets, 2H, J = 9.5, 9.5 $H_z$); 7.45 (2 doublets, 2H, J = 10.0, 10.0 $H_z$); 7.80 (d, 1H, J=11 $H_z$), 7.89 (d, 1H, J=9.5 $H_z$); 9.45 (d, 1H, J=10 $H_z$).

Molecular Formula by
High Resolution Mass Spec.
$C_{32}H_{34}N_4O_3$ = M
$C_{32}H_{35}N_4O_3$ = $M^{+}1$
Molecular Weight:
Calc. 523.2709
Meas. 523.2700

B. To a stirred solution of 4-n-butylstaurosporine of Example 2A (25 mgs in $CH_2Cl_2$, (1 mL) cooled to 5°C, was added a methylene chloride solution of MCPBA (30 mgs). The reaction was complete in less than 30 seconds and the excess reagent was destroyed by addition of a 0.5% solution of sodium bisulfite ($NaHSO_3$ (20 drops)). The so-formed mixture was then extracted with 2 mL of dilute aqueous sodium bicarbonate and the organic layer was dried ($MgSO_4$) and the product was purified by preparative HPLC (15% $MeOH/CH_2Cl_2$) to give 21 mgs of a mixture of diastereoisomeric N-oxides

Molecular Formula by High Resolution
Mass Spec: $C_{32}H_{35}N_4O_4$ (M+1)
Molecular Weight:
Calculated (M+1) 539.2658
Measured (M+1) 539.2621

Diastereoisomer #A of formula III

PNMR δ ($CDCl_3$): 1.08 (t, 3H, J=5.5 $H_z$); 1.46 (sextet, 2H, J= 5.5 $H_z$); 2.21 (s, 3H); 2.55 (S, 3H); 2.69 (t, 1H, J=9.2 $H_z$); 2.90 (s, 3H); 3.08 (br. mult., 1H); 3.39 (br. mult., 2H); 3.86 (dd, 1H, J=3.9 $H_z$); 5.04 (s, 2H); 5.18 (s, 1H); 6.3 (br. S, 1H, $D_2O$ exchangeable); 6.80 (dd, 1H, J=9, 1.5 $H_z$); 7.25 (d, 1H, J=10 $H_z$); 7.4 (2 doublets, 2H, J=9.5 $H_z$); 7.51 (mult, 2H); 7.83 (d, H, J=9.0 $H_z$); 7.95 (d, 1H, J=10 $H_z$); 9.50 (d, 1H, J =10 $H_z$).

Diastereoisomer B of formula III

PNMR δ ($CDCl_3$): 0.90 (t, 3H) J= 6$H_z$); 1.3 (m, 2H); 2.19 (s, 3H); 2.59 (s, 3H); 2.75 (d, t, 1H, J=1.5, 8Hz); 3.1 (t, 2H, J=6.2 $H_z$); 3.28 (br. s. 2H); 4.19 (br. mult. 1H); 4.88 (s, 1H); 5.03 (s, 2H); 6.20 (br. s. 1H, $D_2O$ exchangeable); 6.83 (dd, 1H, J=1.5, 8 $H_z$); 7.25 (d, 1H, J=9.5 $H_z$); 7.40 (2 doublets, 2H, J=10.5 $H_z$); 7.50 (2 doublets, 2H, J=9.5 $H_z$); 7.81 (d, 1H, J=10$H_z$); 7.98 (d, 1H, J=9.5 $H_z$); 9.48 (d, 1H, J=10 $H_z$).
High Resolution FAB Mass. Spec.:
Molecular Weight:
Calc.: 539.2658
Meas.: 539.2608
Molecular Formula (M+1): $C_{32}H_{35}N_4O_4$

Examples 3-10

Follow the procedures of Examples 1 and 2 except substitute for formaldehyde or n-butanal, an equivalent quantity of the appropriate aldehyde to obtain the corresponding 4'-N-alkyl-N-oxide Staurosporine derivative.

Example 11

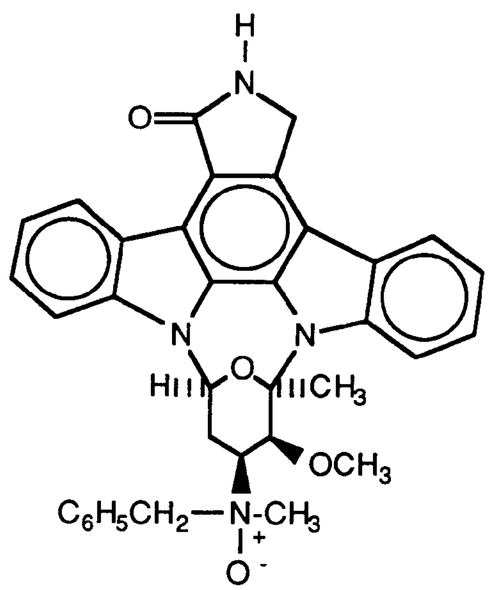

I

A. To a solution of staurosporine (101.8 mgs, 0.21 mmoles) in 5 MLof THF) was added benzyl bromide (40 mgs, 0.23 mmoles) and diisopropylethylamine (60 mgs, 46 mmoles). The so-formed reaction mixture was heated to reflux for three days. The solvent was removed by vacuum distillation, and the so-formed residue was partitioned between water (5 mL) and methylene chloride (5 mL). The organic layer was dried ($MgSO_4$) and concentrated. Product was purified by preparative HPLC (10% MeOH/$CH_2Cl_2$ ) to provide 82 mg of 4'-N-benzylstaurosporine.

PNMR: δ ($CDCl_3$): 2.15 (s, 3H); 2.35 (s, 3H); 2.48 (s, 3H); 2.70 (m, 2H); 3.3 (br. t, 1H, J=10 $H_z$); 3.51 (br. d, 1H, J=10 $H_z$); 3.71 (br. d, 1H, J=10 $H_z$);4.0 (s, 1H); 4.98 (2 doublets, 2H, J=14 $H_z$); 6.62 (dd, 1H, J=7.3, 3$H_z$);7.02 (s, 1H, exchanges with $D_2O$); 7.13 (br. doublet, 2H, J=9.8 $H_z$); 7.2-7.52 (m, 7H); 7.81 (d, 4H, J=10 $H_z$); 7.90 (d, 1H, J=9.5 $H_z$), 9.51 (d, 2H, J=10 $H_z$).

Molecular Formula by High Resolution Mass Spec.: (M+1) = $C_{35}H_{32}N_4O_3$
Molecular Weight:

| | |
|---|---|
| Calculated: | 557.2553 |
| Measured: | 557.2566 |

B. The 4′-N-benzylstaurosporine (150 mgs, 0.27 mmoles) of Example 11A was dissolved in methylene chloride (5 mL) and the so-formed mixture was cooled in an ice bath. A solution of MCPBA (75 mgs) in $CH_2Cl_2$ was add. The reaction was stopped after 1 min by the addition of aq. solvent 0.5% potassium hydrogen sulfite. The organic layer was separated and washed with aqueous dilute sodium bicarbonate and then water. The organic layer was dried (Mg $SO_4$) and product was purified by preparative HPLC 10% MeOH/$CH_2Cl_2$ ) to give 155 mgs of 4′-N-benzyl-N-oxide of staurosporine as a mixture of diastereoisomers.

Molecular Formula by High Resolution Mass Spec.: $C_{35}H_{33}N_4O_3$ (M+1)
Molecular Weight:

| | |
|---|---|
| Calculated: | (M+1) 573.2502 |
| Measured: | (M+1) 573.2512 |

PNMR: δ (DMSO-d6): 2.30 (s, 3H); 2.34 (s, 3H); 2.41 (m, 2H); 2.70 (s, 3H); 3.6 (m, 2H); 4.50 ( 2 doublets, 2H, J=11.1 $H_z$); 5.03 (s, 2H); 5.22 (s, 1H); 7.0 (m, 1H); 7.25-7.9 (complex mul., 10H); 8.12 (t, 2H, J=10.2 $H_z$); 8.2 (s, 1H, $D_2O$ exchangeable); 9.4 (d, 1H, J=10.2$H_z$)

Examples 12-16

Follow the procedure of Example 11 except substitute an equivalent quantity of the appropriately substituted benzyl bromide for benzyl bromide and obtain the corresponding 4′-N-substituted benzyl-N-oxide of staurosporine.

## Claims

1. A compound represented by the formula I

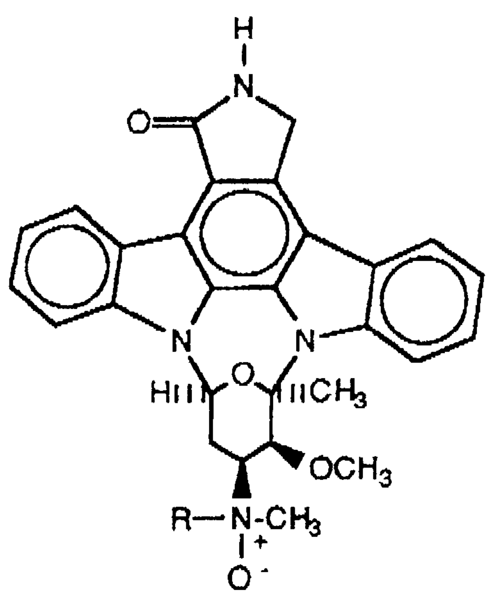

I

wherein R is a straight or branched chain ($C_1$-$C_{10}$) alkyl group or an arylmethyl group and N-oxide stereochemical isomers thereof.

2. A compound of claim 1 wherein R=$CH_3$-

3. A compound of claim 1 wherein R=($C_1$-$C_{10}$) alkyl.

**4.** A compound of claim 1 wherein R=n - $C_4H_9$-.

**5.** A compound of claim 1 wherein R=arylmethyl.

**6.** A compound of claim 1 wherein R=$C_6H_5CH_2$-.

**7.** A pharmacautical composition comprising a therapeutically effective amount of a compound of claim 1 and a pharmaceutically acceptable carrier.

**8.** A method of treating susceptible tumors in mammals which comprises administering to a mammal in need of such treating an antitumor amount of a compound of claim 1 effective for such purpose or a pharmaceutical composition containing said effective amount of said compound.

**9.** A method of claim 8 wherein the susceptible tumors are P388 lymphotic leukemia and HL-60 promyelocytic leukemia tumors.

**10.** A method of treating psoriasis in mammals which comprises administering to a mammal in need of such treating an antipsoriatically effective amount of a compound of claim 1 or a pharmaceutical composition containing said effective amount of said compound.

European Patent Office

# EUROPEAN SEARCH REPORT

Application Number

EP 92 30 9053

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A<br><br>A,D | EP-A-0 296 110 (CIBA-GEIGY AG)<br>* claims 1,24; examples 1-42 *<br>& AU-D-1 757 188<br>--- | 1,7 | C 07 D 498/22<br>A 61 K 31/55 //<br>(C 07 D 498/22<br>C 07 D 311:00<br>C 07 D 273:00<br>C 07 D 209:00<br>C 07 D 209:00<br>C 07 D 209:00 ) |
| A | WO-A-9 109 034 (SCHERING CORP.)<br>* claims 1,5,6; examples 9,16 *<br>--- | 1,7 | |
| A | EP-A-0 383 919 (KYOWA HAKKO KOGYO CO., LTD.)<br>* page 1, paragraph 1 *<br>--- | 1,7 | |
| A | PATENT ABSTRACTS OF JAPAN vol. 10, no. 31 (C-327)(2088) 6 February 1986<br>& JP-A-60 185 719 ( AJINOMOTO K.K. ) 21 September 1985<br>--- | 1,7 | |
| A,D | US-A-4 107 297 (S. OMURA ET AL.)<br>----- | | |
| | | | **TECHNICAL FIELDS SEARCHED (Int. Cl.5)** |
| | | | C 07 D |

The present search report has been drawn up for all claims

| Place of search | Date of completion of the search | Examiner |
|---|---|---|
| BERLIN | 08-12-1992 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P0401)